(19) Europäisches Patentamt
European Patent Office
Office européen des brevets

(11) Publication number : **0 432 630 B1**

(12) # EUROPEAN PATENT SPECIFICATION

(45) Date of publication of patent specification : **16.03.94 Bulletin 94/11**

(51) Int. Cl.$^5$ : **A61K 31/47**

(21) Application number : **90123375.9**

(22) Date of filing : **06.12.90**

(54) **Antitumor agent.**

(30) Priority : **13.12.89 JP 321503/89**

(43) Date of publication of application : **19.06.91 Bulletin 91/25**

(45) Publication of the grant of the patent : **16.03.94 Bulletin 94/11**

(84) Designated Contracting States : **BE CH DE ES FR GB IT LI NL SE**

(56) References cited :
**US-A- 4 767 626**
**CHEMICAL ABSTRACTS, vol. 107, no. 21, 23rd November 1987, page 799, abstract no. 198705c, Columbus, Ohio, US; H. ISHII et al.: "Establishment of generally applicable method for phenolic antileukemic benzo[c]phenanthridine alkaloids using intramolecular basic acylation. Synthesis of fagaronine and structural establishment of oxyterihanine", & TENNEN YUKI KAGOBUTSU TORONKAI KOEN YOSHISHU, 1986, 28th, 457-64**
**JOURNAL OF PHARMACEUTICAL SCIENCES, vol. 61, no. 11, November 1972, pages 1858-1859; W.M. MESSMER et al.: "Fagaronine, a new tumor inhibitor isolated from Fagara zanthoxyloides Lam.(Rutaceae)"**

(56) References cited :
**CHEM. PHARM. BULL., vol. 33, no. 4, 1985, pages 1763-1765; M. HANAOKA et al.: "Synthesis of fagaridine, a phenolic benzo[c]phenanthridine alkaloid"**
**FITOTERAPIA, vol. 58, no. 2, 1987, pages 123-126; S.K. ADESINA: "Further new constituents of Zanthoxylum leprieurii"**

(73) Proprietor : **NIPPON KAYAKU KABUSHIKI KAISHA**
**11-2, Fujimi 1-chome Chiyoda-ku**
**Tokyo 102 (JP)**

(72) Inventor : **Hanaoka, Miyoji**
**149-2, Kubo-1-chome**
**Kanazawa-shi (JP)**
Inventor : **Ekimoto, Hisao**
**11-1-803, Shimo-2-chome**
**Kita-ku, Tokyo (JP)**
Inventor : **Kobayashi née Matsumura, Fumiko**
**13-9-602, Furuishiba-3-chome**
**Koto-ku, Tokyo (JP)**
Inventor : **Irie, Yukio**
**2 3, Shimokawamachi**
**Maebashi-shi (JP)**
Inventor : **Takahashi, Katsutoshi**
**10-8-512 Kamiya-3-chome**
**Kita-ku, Tokyo (JP)**

(74) Representative : **Türk, Dietmar, Dr. rer. nat. et al**
**Türk, Gille, Hrabal, Leifert Patentanwälte**
**Brucknerstrasse 20**
**D-40593 Düsseldorf (DE)**

**EP 0 432 630 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to compounds for medical use and pharmaceutical compositions which are useful for the prevention and treatment of malignant tumor in warm-blooded animals.

Nowadays, alkylating agents, nucleic acid metabolism antagonists, antibiotics, plant alkaloids and the like have been used as chemotherapeutic drugs for patients with tumors but these drugs do not have satisfactory efficacy yet.

It is also known that malignant tumors such as gastric cancer, lung cancer, etc. or diseases associated with hematopoietic organ, e.g., leukemia, etc. cause disseminated intravascular coagulation syndrome due to cancer or hypoxia, etc. which result in stimulation of endogenous and exogenous blood coagulation system [SOGO RINSHO (general clinic), 34, 2360-2364, 1985]. Accordingly, conventional chemotherapeutic agents are not effectively taken up into cancer cells to exhibit their activity.

It has thus been desired to develop antitumor agents which act on the blood coagulation system to show preventive effect of blood coagulation and at the same time, exhibit an antitumor activity.

The present invention provides compounds for medical application or pharmaceutical compositions.

The compounds for medical application or pharmaceutical compositions of the present invention are useful for the treatment of malignant tumor of warm-blooded animals including human and exhibit an excellent antitumor activity not only against blood tumors but also against solid tumors.

As a result of extensive studies, the present inventors have found that 7-hydroxy-8-methoxy-5-methyl-2,3-methylenedioxybenzo[c]phenanthridinium acidic quaternary salts shown by formula A described below have both the preventive effect on platelet aggregation and the antitumor activity and have thus reached the present invention. That is, the present invention provides 7-hydroxy-8-methoxy-5-methyl-2,3-methylenedioxybenzo[c]-phenanthridinium acidic quaternary salts shown by formula A described below as novel drugs:

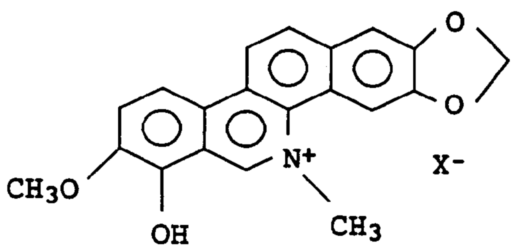

(A)

[wherein $X^-$ is an acid residue]. Compound A is known in the art as "fagaridine" (INN).

Pharmaceutical activities of other benzo (c) phenanthridine alkaloids chemically related to fegaridine are known in the art (J. Pharm. Sci., vol. 61(11), 1972, 1858-1859). However no efforts have been made so far to investigate possible biological effects of fagaridine.

DETAILED DESCRIPTION OF THE INVENTION

Where the compound of formula A is used as a drug, the compound may usually be prepared into a pharmaceutical composition comprising the compound of formula A and pharmaceutical additives, and the composition is provided for use. An effective dose of the compound of formula A is administered to warmblooded animal with tumor, whereby growth of tumor can be inhibited and tumor can be treated.

It is considered that when administered, the compound of the present invention would be dissociated in vivo as shown by the following formula A'.

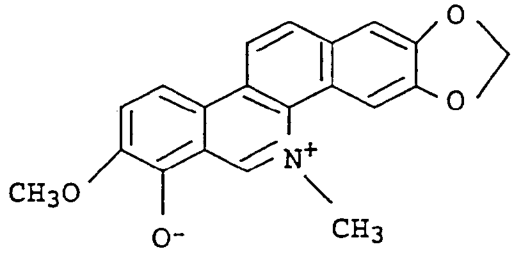

(A')

Examples of the acid residue shown by $X^-$ in the compound of formula A which is used in the present invention include inorganic acid residues such as halogen, e.g., chlorine ions, bromine ions, iodine ions, fluorine ions ; nitric ions; phosphatic ions; sulfuric ions; and the like; residues of organic acids such as dimethyl sulfate, diethyl sulfate, etc.

7-Hydroxy-8-methoxy-5-methyl-2,3-methylenedioxybenzo[c]phenanthridinium of the present invention can be prepared and obtained by the process described in, e.g., Chem. Pharm. Bull, 33, 1763-1765 (1985).

The compounds of formula A in accordance with the present invention have an excellent antitumor activity as will be later shown in pharmacological tests. The compounds exert a remarkable growth inhibition activity on various tumor cells cultured. Furthermore, the compounds of the present invention inhibit growth of tumor in various animals with tumor and exhibit a prolongation of increased life span. In addition, the compounds inhibit platelet aggregation due to platelet activating factor and inhibit metastasis of tumor. Therefore, the antitumor composition of the present invention is effective for solid tumors of human, for example, gastric cancer, liver cancer, rectal cancer, lung cancer, etc.; and for blood diseases such as leukemia, Hodgkin's disease, etc.

When the compounds of formula A in accordance with the present invention are used as drugs, the compounds may be prepared into pharmaceutical preparations and the preparations may be applied in various conventional manners. That is, the preparations may be applied parenterally, orally, intrarectally, etc. The preparations may take the form of an injection, powder, a granulate, a tablet, a suppository, etc. In preparing the pharmaceutical composition, a variety of auxiliary agents used in drugs, namely, carrier and other aids, for example, a stabilizer, an antiseptic, a pain killer, an emulsifying agent, etc. may be used, if necessary and desired.

In the composition, the content of the compound shown by formula A may be varied over a wide range depending upon form of preparation, but the composition may contain generally in an amount of 0.01 to 100% by weight, preferably 0.1 to 50% by weight, of the compound shown by formula A. The balance is a carrier and other auxiliary agents used for conventional drug compositions.

A dose of the compound of formula A varies depending on condition, etc., but is generally approximately 50 to 500 mg per day for adult.

Hereinafter pharmacological tests and examples are shown below but the present invention is not deemed to be limited thereto.

Pharmacological Tests:

As a test drug, 7-hydroxy-8-methoxy-5-methyl-2,3-methylenedioxybenzo[c]phenanthridinium chloride was used.

1. Growth inhibition test on tumor cells in vitro

Experimental conditions for each cell are shown in Table 1. After the test drug was exposed for 2 to 4 days on each cell cultured at 37°C for 24 hours in 5% $CO_2$ under the conditions, the cells were stained with 0.05% Methylene Blue. The pigment was extracted from the stained cells. A growth inhibition rate of the cell was determined based on absorbance of the extracted pigment at 660 nm and 50% growth inhibitory concentration ($IC_{50}$) was calculated.

Table 1. Culture Condition for Various Tumor Cells

| Cell | Count of Cell Inoculated | Time for Incubation |
| --- | --- | --- |
| Hela $S_3$ | $7.5 \times 10^3$/ml | 72 hours |
| Colon 26 | $7.5 \times 10^3$/ml | 65 hours |
| SW1116 | $1.5 \times 10^4$/ml | 96 hours |
| Li-7 | $1.5 \times 10^4$/ml | 48 hours |
| HepG2 | $1.0 \times 10^4$/ml | 96 hours |
| LL | $4.0 \times 10^3$/ml | 72 hours |
| PC-3 | $1.0 \times 10^4$/ml | 72 hours |
| B16 | $7.5 \times 10^3$/ml | 72 hours |

The results are shown in Table 2. As is obviously noted from Table 2, the test drug inhibited the growth of various tumor cells.

Table 2. Cell Growth Inhibition Activity Against Various Tumor Cells

| Kind of Cell | $IC_{50}$ (μg/ml) |
| --- | --- |
| Hela $S_3$ | 0.32 |
| Colon 26 | 0.038 |
| SW1116 | 0.25 |
| Li-7 | 0.12 |
| HepG2 | 0.059 |
| LL | 0.044 |
| PC-3 | 0.21 |
| B16 | 0.057 |

2. Antitumor Activity against Tumors Cell in vivo

Antitumor activity in vivo (1)

Three kinds of mouse leukemia P388, fibrosarcoma M5076 and colon cancer C26 were used. P388 ($10^6$/mouse) and C26 ($10^5$/mouse) were intraperitoneally or subcutaneously transplanted to female $CDF_1$ mice (5 mice per one group) of 6 weeks age. M5076 ($10^6$/mouse) was subcutaneously transplanted to female C57BL/6 mice (5 mice per one group) of 6 weeks age. The test drug was dissolved in 20% dimethylsulfoxide upon use; the solution was intraperitoneally administered to the P388 group and the M5076 group once a day for consecutive

5 days from the next day following the transplantation of tumor. The solution was intraperitoneally administered to the C26 group once a day for consecutive 5 days from Day 5 after the transplantation of tumor. An antitumor activity was judged by a rate (T/C %) of the median in the number of survival dates in each group to that in the control group.

The results are shown in Tables 3, 4 and 5. As is clear from Tables 3, 4 and 5, the compound exerted a potent antitumor activity on the three kinds of tumor.

Table 3. Effect on Leukemia P388

| | Dose (mg/kg/day) | Median Survival Time (day) | Effect (T/C %) |
|---|---|---|---|
| Test Drug | 25 | 22.5 | 223 |
| | 12.5 | 16.5 | 163 |
| | 6.25 | 14.8 | 147 |
| | 3.13 | 13.5 | 134 |
| Group administered with physiological saline | - | 10.1 | 100 |

Table 4. Effect on Fibrosarcoma M5076

| | Dose (mg/kg/day) | Median Survival Time (day) | Effect (T/C %) |
|---|---|---|---|
| Test Drug | 25 | 28.5 | 154 |
| | 12.5 | 20.5 | 111 |
| | 6.25 | 20.5 | 111 |
| Group administered with physiological saline | - | 18.5 | 100 |

Table 5. Effect on Colon Cancer C26

| | Dose (mg/kg/day) | Median Survival Time (day) | Effect (T/C %) |
|---|---|---|---|
| Test Drug | 50 | 42.5 | 139 |
| | 25 | 31.3 | 103 |
| | 12.5 | 33.5 | 110 |
| Group administered with physiological saline | - | 30.5 | 100 |

Antitumor Activity in vivo (2)

(a) Mouse colon tumor

Mouse colon tumor C26 ($10^5$/mouse) was subcutaneously transplanted to female $CDF_1$ mice (3 mice per one group) of 6 weeks age. The test drug was intravenously administered once a day for consecutive 5 days from Day 7 after transplantation of the tumor at a dose of 50 mg/kg; or intravenously administered 3 times once a day every 4 other days in a dose of 75 mg/kg.

(b) Mouse lung tumor

Mouse lung tumor LL ($10^6$/mouse) was subcutaneously transplanted to male $BDF_1$ mice (3 mice per one group) of 6 weeks age. The test drug was intravenously administered 3 times once a day every 4 other days at a dose of 60 mg/kg on Day 8 after the transplantation.

(c) Human liver tumor

Human liver tumor Li-7 (fragment) was subcutaneously transplanted to female nude BALB-c/nu-A mice (3 mice per one group) of 6 weeks age. The test drug was intravenously administered 3 times once a day every 4 other days at a dose of 60 mg/kg on Day 8 after the transplantation.

In (a), (b) and (c) described above, the antitumor activity was determined by a ratio (T/C %) of the administered group to the control group in the number of day until a size of the tumor became 10 times that at the time when the administration started and increased life span of mice (T/C %). The results are shown in Table 6. The results reveal that the compound inhibit growth of C26, LL and Li-7 tumors and its increased life span shows as high as 130% or more in the mice with any of the tumors.

Table 6

| Tumor Cell | Test Compound Dose (mg/kg) | Schedule | Rate of tumor growth delay (%) | Increased Life Span (T/C %) |
|---|---|---|---|---|
| C26 | 50 | gld x 5 | 219 | 135 |
| C26 | 75 | g4d x 3 | 203 | 135 |
| LL | 75 | g4d x 3 | 118 | 152 |
| Li-7 | 60 | g4d x 3 | 253 | 137 |

3. Inhibition activity on platelet aggregation

Platelet-rich plasma (PRP) was collected from Japanese albino rabbits (weighing 3-4 kg). As a platelet aggregation inducer, platelet activating factor (PAF) was used in a final concentration of $10^{-7}$ M. The test drug was added to PRP. After incubation for a definite period of time, PAF was added to cause platelet aggregation reaction. The reaction was terminated with EDTA. After the centrifugation, the supernatant was removed to obtain the platelet pellet. Destilled water was added to the platelet precipitates, whereby serotonin remained in platelet was reacted with orthophthalaldehyde reagent to form sertonin-orthophthalaldehyde condensate. The condensate was measured at an excited wavelength of 360 nm and at a measurement wavelength of 475 nm. Anti-PAF activity of the test drug was determined by the following equation.

$$\text{Serotonin release inhibition rate (\%)} = \frac{\text{(Test drug plus PAF) value} - \text{PAF value}}{\text{Blank value} - \text{PAF value}} \times 100$$

The result is shown in Table 7. As is clear from Table 7, the test drug showed a potent inhibition activity on platelet aggregation.

Table 7. **Inhibition Activity on Platelet Aggregation**

| | Concentration (μg/ml) | Inhibition Rate (%) |
|---|---|---|
| Test Drug | 100 | 107 |
| | 50 | 107 |
| | 25 | 85.5 |
| | 12.5 | 48.6 |
| Physiological saline | - | 0.0 |

4. Inhibition Activity of Tumor Metastasis in vivo

The test drug was intraperitoneally administered to male C57BL/6 mice of 5 weeks age at a dose of 25 or 50 mg/kg. Fifteen minutes after, highly metastatic mouse melanoma B16BL6 ($10^5$/mouse) was transplanted to mice through the tail vein. After mice were bred to death on Day 14 after the transplantation, the number of metastasis of BL6 into the lung was visually observed.

As is clear from Table 8, the test drug inhibited metastasis of melanoma into the lung.

Table 8. Inhibition Activity of Tumor Metastasis in vivo

| | Dose (mg/kg) | Number of Metastatic Node (mean + S. E.) |
|---|---|---|
| Test Drug | 50 | 76.9 ± 13.6 |
| | 25 | 55.8 ± 15.7 |
| Physiological saline | - | 111.9 ± 19.8 |

5. Acute toxicity

The test drug was intravenously administered to female $CDF_1$ mice of 6 weeks age. The mice survived a dose of 100 mg/kg without showing any lethal toxicity.

The foregoing results reveal that 7-hydroxy-8-methoxy-5-methyl-2,3-methylenedioxybenzo[c]phenanthridinium chloride of the present invention exhibits an antitumor activity and inhibition activity on platelet aggregation, and is thus expected as a useful agent for the treatment of tumors.

Example 1 (pharmaceutical composition)

After 1 g of the test drug used in the experiments described above, 1 g of polysorbate and 1 g of Macrogol 400 were dispersed and dissolved in 100 g of distilled water for injection. After filtering through a membrane filter, the solution was separately charged in ampoules and lyophilized in a conventional manner to give a preparation for injection containing 50 mg of the compound per ampoule.

**Claims**

1. A pharmaceutical composition comprising as an effective ingredient a 7-hydroxy-8-methoxy-5-methyl-2,3-methylenedioxybenzo[c]phenanthridinium acidic quaternary salt represented by formula A:

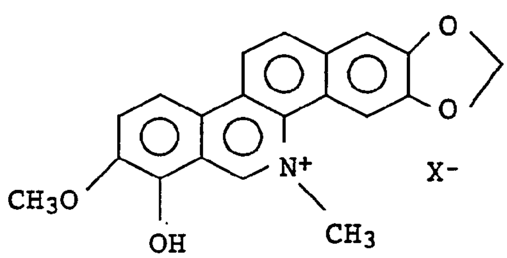

(A)

[wherein $X^-$ is an acid residue] and pharmaceutically acceptable additives.

2. A composition according to claim 1, wherein said acidic quaternary salt is a halogenoacid salt.

3. A composition according to claim 1, wherein said acidic quaternary salt is chloride.

4. A composition according to claim 1 for the growth inhibition, metastasis inhibition and treatment of malignant tumors in warm-blooded animal.

5. Use of a compound represented by formula A:

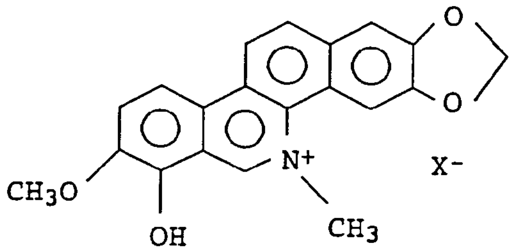

(A)

[wherein $X^-$ is an acid residue] for preparing a pharmaceutical composition for the treatment, growth inhibition and metastasis inhibition of malignant tumor.

6. A 7-hydroxy-8-methoxy-5-methyl-2,3-methylenedioxybenzo[c]phenanthridinium acidic quaternary salt represented by formula A:

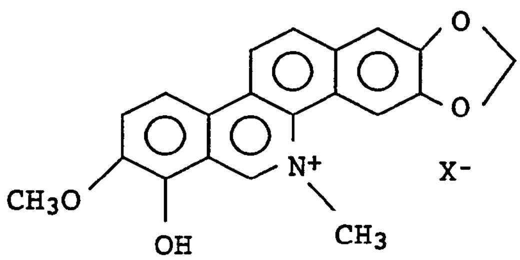

(A)

[wherein X⁻ is an acid residue] for use as a medicament.

7. Use of a compound according to claim 5, wherein $X^-$ is a halogen ion.

8. Use of a compound according to claim 5 or 7, wherein $X^-$ is a chloride ion.

9. Compound of claim 6, wherein $X^-$ is a halogen ion, for use as a medicament.

10. Compound of claim 6 or 9, wherein $X^-$ is a chloride ion, for use as a medicament.

## Patentansprüche

1. Pharmazeutische Zusammensetzung, die als wirksame Komponente (Wirkstoff) ein quaternäres 7-Hydroxy-8-methoxy-5-methyl-2,3-methylendioxybenzo[c]phenanthridiniumsäuresalz der Formel (A)

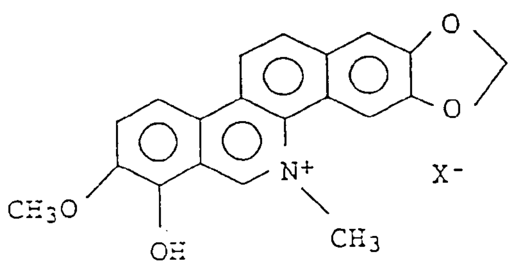

(A)

worin $X^-$ für einen Säurerest steht,
und pharmazeutisch akzeptable Zusätze enthält.

2. Zusammensetzung nach Anspruch 1, worin das quaternäre Säuresalz ein Halogensäuresalz ist.

3. Zusammensetzung nach Anspruch 1, worin das quaternäre Säuresalz ein Chlorid ist.

4. Zusammensetzung nach Anspruch 1 zur Hemmung des Wachstums und der Metastase sowie zur Behandlung von malignen Tumoren bei einem Warmblüter.

5. Verwendung einer Verbindung der Formel (A)

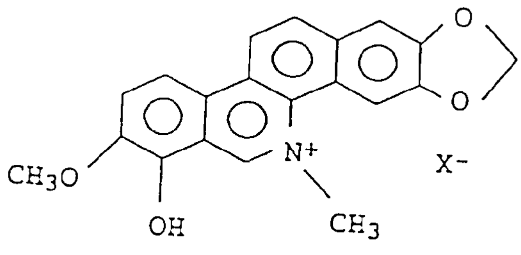

(A)

worin X⁻ für eine Säurerest steht,
zur Herstellung einer pharmazeutischen Zusammensetzung für die Behandlung, zur Hemmung des Wachstums und zur Hemmung der Metastasierung eines malignen Tumors.

6. Quaternäres 7-Hydroxy-8-methoxy-5-methyl-2,3-methylendioxybenzo[c]phenanthridinium-Säuresalz der Formel A:

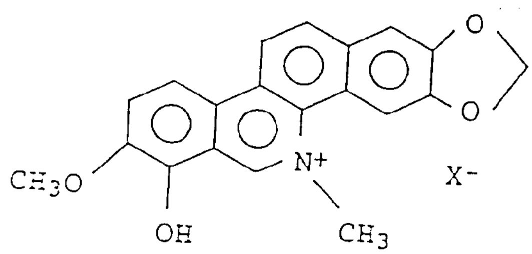

(A)

worin $X^-$ für einen Säurerest steht,
für die Verwendung als Arzneimittel.

7. Verwendung einer Verbindung nach Anspruch 5, worin $X^-$ ein Halogenion bedeutet.

8. Verwendung einer Verbindung nach Anspruch 5 oder 7, worin $X^-$ ein Chloridion bedeutet.

9. Verbindung nach Anspruch 6, worin $X^-$ ein Halogenion bedeutet, für die Verwendung als Arzneimittel.

10. Verbindung nach Anspruch 6 oder 9, worin $X^-$ ein Chloridion bedeutet, für die Verwendung als Arzneimittel.

## Revendications

1. Composition pharmaceutique comprenant en tant que principe actif un sel quaternaire acide de 7-hydroxy-8-méthoxy-5-méthyl-2,3-méthylènedioxybenzo[c]phénanthridinium représenté par la formule A :

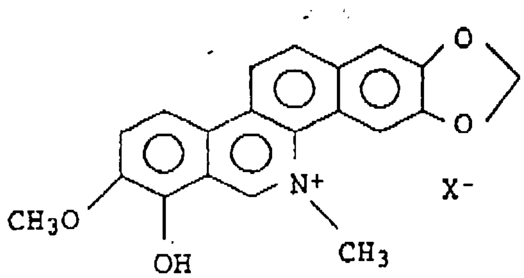

(A)

[dans laquelle $X^-$ est un reste acide] et des additifs pharmaceutiquement acceptables.

2. Composition selon la revendication 1, dans laquelle ledit sel quaternaire acide est un sel d'halogénoacide.

3. Composition selon la revendication 1, dans laquelle ledit sel quaternaire acide est un chlorure.

4. Composition selon la revendication 1 pour l'inhibition de croissance, l'inhibition de métastases et le traitement de lumeurs malignes chez des animaux à sang chaud.

5. Utilisation d'un composé représenté par la formule A :

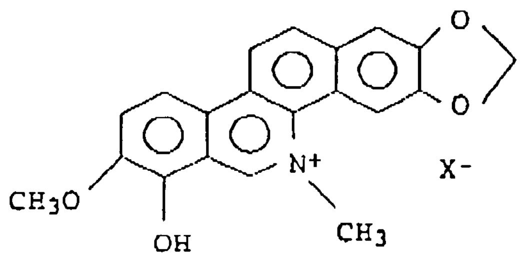

(A)

[dans laquelle $X^-$ est un reste acide] pour la préparation d'une composition pharmaceutique pour le traitement, l'inhibition de croissance et l'inhibition de métastases de tumeurs malignes.

6. Un sel quaternaire acide de 7-hydroxy-8-méthoxy-5-méthyl-2,3-méthylènedioxybenzo[c]phénanthridinium représenté par la formule A :

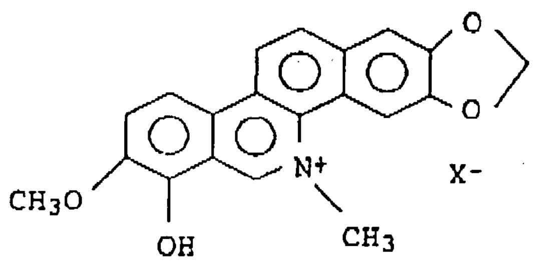

(A)

[dans laquelle $X^-$ est un reste acide] pour l'utilisation en tant que médicament.

7. Utilisation d'un composé selon la revendication 5, dans lequel $X^-$ est un ion halogène.

8. Utilisation d'un composé selon la revendication 5 ou 7, dans lequel $X^-$ est un ion chlorure.

9. Composé selon la revendication 6, dans lequel $X^-$ est un ion halogène, pour l'utilisation en tant que médicament.

10. Composé selon la revendication 6 ou 9, dans lequel $X^-$ est union chlorure, pour l'utilisation en tant que médicament.